# EUROPEAN PATENT APPLICATION

(11) **EP 3 646 771 A1**
(43) Date of publication of application: **06.05.2020**
(21) Application number: 18822911.6
(22) Date of filing: 28.05.2018
(51) Int. Cl.: A61B 1/045, A61B 1/00, A61B 1/04, G02B 5/20, H04N 5/225, H04N 5/232, H04N 9/09

(54) **SURGICAL IMAGING SYSTEM AND SIGNAL PROCESSING DEVICE FOR SURGICAL IMAGE**

(30) Priority: 26.06.2017 JP 2017124074
(71) Applicant: Sony Corporation, Tokyo 108-0075 (JP)
(72) Inventor: KASHIMA, Koji, Tokyo 108-0075 (JP); HAYASHI, Tsuneo, Tokyo 108-0075 (JP); TAKAHASI, Kenji, Tokyo 108-0075 (JP); MIZUKURA, Takami, Tokyo 108-0075 (JP); KIKUCHI, Daisuke, Tokyo 108-0075 (JP)
(74) Representative: D Young & Co LLP
(86) International application number: PCT/JP2018/020326
(87) International publication number: WO 2019/003751

(57) **Abstract**

To improve resolution of an image in a case of imaging with an image sensor having light receiving sensitivity in a long wavelength region.

A surgical imaging system according to the present disclosure includes a first image sensor that has light receiving sensitivity in a wavelength region of visible light and images a surgical site, a second image sensor that has light receiving sensitivity in a wavelength region of visible light and near-infrared light and images the surgical site, and a signal processing device that performs a process for displaying a first image imaged by the first image sensor and a second image imaged by the second image sensor.

## Description

### TECHNICAL FIELD

The present disclosure relates to a surgical imaging system and a signal processing device of a surgical image.

### BACKGROUND ART

Conventionally, for example, following Patent Document 1 discloses a configuration in which a Si-based CCD, a CMOS camera and the like are used as a first imaging means, and an InGaAs camera, a germanium camera, a vidicon camera and the like are used as a second imaging means, the second imaging means not having sensitivity to a wavelength of visible light.

### CITATION LIST

### PATENT DOCUMENT

Patent Document 1: Japanese Patent Application Laid-Open No. 2013-162978

### SUMMARY OF THE INVENTION

### PROBLEMS TO BE SOLVED BY THE INVENTION

However, an image sensor using indium gallium arsenide (InGaAs) generally has lower resolution than resolution of a silicon-based image sensor. For this reason, in the technology disclosed in Patent Document described above, for example, in a case of observing a surgical site, it is difficult to obtain a high-resolution image such as a visible light image because of low resolution of the InGaAs camera.

Therefore, it has been desired to improve the resolution of the image in a case of imaging with an image sensor having light receiving sensitivity in a long wavelength region.

### SOLUTIONS TO PROBLEMS

The present disclosure provides a surgical imaging system including a first image sensor that has light receiving sensitivity in a wavelength region of visible light and images a surgical site, a second image sensor that has light receiving sensitivity in a wavelength region of visible light and near-infrared light and images the surgical site, and a signal processing device that performs a process for displaying a first image imaged by the first image sensor and a second image imaged by the second image sensor.

Furthermore, the present disclosure provides a signal processing device of a surgical image performing a process for synthesizing to display a first image imaged by a first image sensor that has light receiving sensitivity in a wavelength region of visible light and images a surgical site and a second image imaged by a second image sensor that has light receiving sensitivity in a wavelength region of visible light and near-infrared light and images the surgical site.

### EFFECTS OF THE INVENTION

According to the present disclosure, it becomes possible to improve the resolution of the image in a case of imaging with the image sensor having the light receiving sensitivity in the long wavelength region.

Note that, the effect described above is not necessarily limited, and it is also possible to obtain any one of the effects described in this specification or another effect which may be grasped from this specification together with or in place of the effect described above.

### BRIEF DESCRIPTION OF DRAWINGS

Fig. 1 is a block diagram illustrating a configuration of a system according to an embodiment of the present disclosure.
Fig. 2 is a characteristic diagram illustrating spectral sensitivity characteristics of an Si image sensor and an InGaAs image sensor.
Fig. 3 is a schematic diagram illustrating an example of the number of sensor pixels of the Si image sensor and the InGaAs image sensor.
Fig. 4A is a schematic diagram illustrating a Bayer method of combining each pixel with any one of color filters of three colors of red, green, and blue (RGB).
Fig. 4B is a schematic diagram illustrating an example in which a plurality of color filters which transmits a specific wavelength region is applied for each pixel in an InGaAs image sensor.
Fig. 4C is a schematic diagram illustrating an example in which a plurality of color filters which transmits a specific wavelength region is applied for each pixel in the InGaAs image sensor.
Fig. 4D illustrates an example in which a red filter is applied to the InGaAs image sensor.
Fig. 5 is a schematic diagram illustrating an example in which a three-plate system using dedicated Si image sensors for R, G, and B in combination with a dichroic mirror is employed.
Fig. 6 is a characteristic diagram illustrating transmissivity of living tissue.
Fig. 7 is a schematic diagram illustrating an image obtained by imaging while combining the InGaAs image sensor with a filter which transmits a wavelength region from 1400 to 1500 nm, and a visible light image obtained by imaging with the Si image sensor.
Fig. 8 is a schematic diagram illustrating an example in which a blood vessel may be recognized through a fat portion in a case where a subject in which the fat portion covers an organ including the blood vessel is imaged.
Fig. 9 is a flowchart illustrating a process performed in a system according to this embodiment.
Fig. 10 is a schematic diagram illustrating a synthesizing processor and a peripheral configuration thereof.
Fig. 11A is a schematic diagram illustrating an optical system of an imaging device.
Fig. 11B is a schematic diagram illustrating the optical system of the imaging device.
Fig. 11C is a schematic diagram illustrating the optical system of the imaging device.

### MODE FOR CARRYING OUT THE INVENTION

A preferred embodiment of the present disclosure is hereinafter described in detail with reference to the accompanying drawings. Note that, in this specification and the drawings, the components having substantially the same functional configuration are assigned with the same reference sign and the description thereof is not repeated.

Note that the description is given in the following order.
1. Outline of the present disclosure
2. Configuration example of system
3. Alignment and synthesis of images obtained from two image sensors
4. Extraction of useful information image
5. Process performed in surgical imaging system according to this embodiment
6. Configuration example of optical system

### 1. Outline of the present disclosure

An imaging device is widely used in order to image the inside of a human body. However, it is actually difficult to correctly determine a state of an organ and the like in the human body only with a normal visible light image. For this reason, it is assumed to mount an InGaAs image sensor sensitive to a near-infrared wavelength region on a surgical imaging system in order to make it easy to visually recognize the inside of the human body, for example, blood vessels and fat regions in deep sites. However, at present, the InGaAs image sensor has a problem of a large pixel size and low resolution as compared with an Si image sensor used in imaging of a conventional visible light image.

Therefore, in the present disclosure, in order to compensate for the low resolution of the InGaAs image sensor, a surgical imaging system utilizing two imaging elements of the InGaAs image sensor and the Si image sensor is devised. The InGaAs image sensor according to the present disclosure is an image sensor sensitive to a continuous wavelength region from a visible light region to the near-infrared wavelength region and may also obtain a signal in a visible region. That is, in the present disclosure, a maximum value λ1max of a wavelength region of the Si image sensor and a minimum value of a wavelength region of the InGaAs image sensor satisfy a relationship that λ2min is "λ1max>λ2min".

Especially, in the surgical imaging system, high resolution is required for an image obtained by imaging. According to the present disclosure, in a case where the InGaAs image sensor sensitive to the visible light region to the near-infrared wavelength region is used, it is possible to compensate for relatively low resolution by the InGaAs image sensor by a high-resolution Si image sensor by combining the Si image sensor. Therefore, it is possible to easily visually recognize the blood vessels and fat regions in the deep sites as described above by light receiving sensitivity in the near-infrared wavelength region, and it is possible to obtain a high-resolution image by the Si image sensor. Furthermore, by utilizing correlation between image information of the visible light image imaged by the Si image sensor and image information of the visible light image imaged by the InGaAs image sensor, alignment between the images of both the Si image sensor and the InGaAs image sensor may be performed. Moreover, by synthesizing the image information of the visible light image imaged by the Si image sensor and the visible light image and infrared light image imaged by the InGaAs image sensor, the information obtained from both the sensors may be visually recognized efficiently.

### 2. Configuration example of system

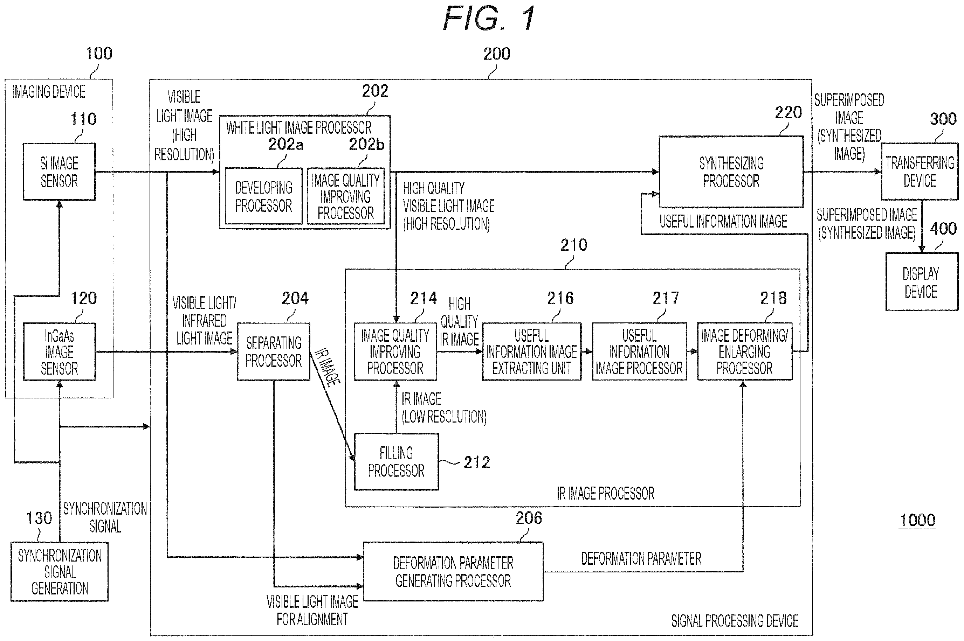

Fig. 1 is a block diagram illustrating a configuration of a surgical imaging system 1000 according to an embodiment of the present disclosure. The surgical imaging system 1000 observes, for example, the blood vessels, fat regions, fluorescent reactions (fluorescent substance and autofluorescence) and the like in the human body, and is applicable to, for example, an endoscope system, a video microscope system and the like. As illustrated in Fig. 1, the surgical imaging system 1000 includes an imaging device 100, a signal processing device 200, a transmitting device 300, and a display device 400.

The imaging device 100 includes two imaging elements of an Si image sensor 110 and an InGaAs image sensor 120. The Si image sensor 110 and the InGaAs image sensor 120 image the same subject. For this reason, the Si image sensor 110 and the InGaAs image sensor 120 are synchronized by a synchronization signal generated by a synchronization signal generating unit 130. The synchronization signal generating unit 130 may be provided in the imaging device 100. At the time of imaging, simultaneous imaging by the Si image sensor 110 and the InGaAs image sensor 120 or frame sequential imaging by time division is performed. Note that, signal processing and display are normally performed while imaging in real time, but the signal processing and display may also be performed when reproducing recorded image data.

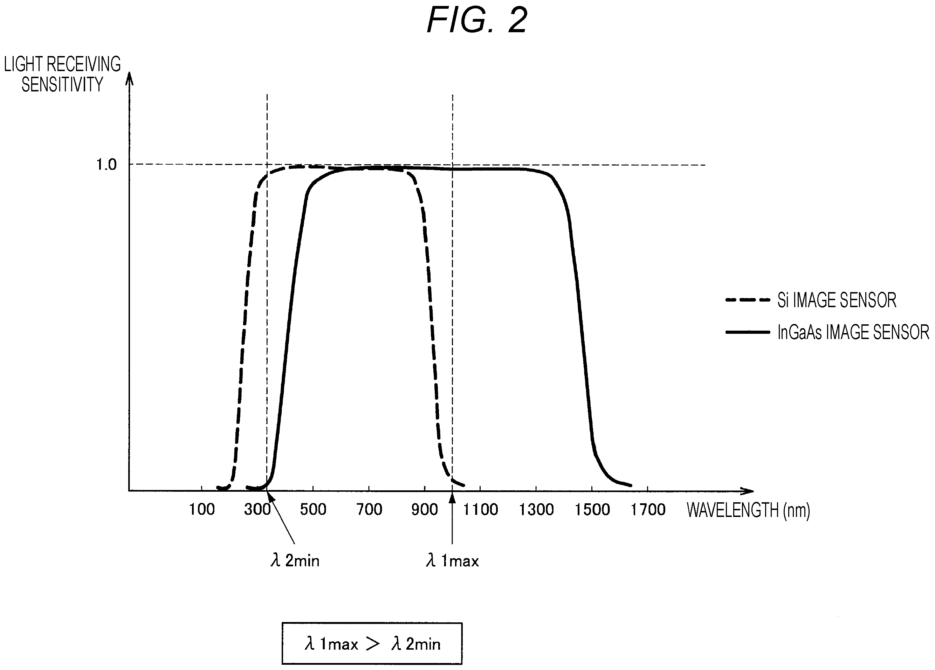

Fig. 2 is a characteristic diagram illustrating spectral sensitivity characteristics of the Si image sensor 110 and the InGaAs image sensor 120. As illustrated in Fig. 2, the InGaAs image sensor 120 has wide band light receiving sensitivity including from the visible light region to a long wavelength region. More specifically, the InGaAs image sensor 120 is sensitive to a continuous wavelength region approximately from 350 nm to 1500 nm. On the other hand, the Si image sensor 110 has light receiving sensitivity in the visible light region. Therefore, a high-resolution visible light image is obtained by the Si image sensor 110, and a visible light image and an infrared light image are obtained by the InGaAs image sensor 120. Note that, hereafter, the visible light image and the infrared light image are referred to as a visible light/infrared light image. Furthermore, the infrared light image is also referred to as an IR image.

As a light source used when imaging, a light source capable of emitting a wide band from a visible region to the near-infrared wavelength region may be employed. Furthermore, in a case where the near-infrared wavelength region is used for fluorescence observation, a narrow wavelength light source for exciting fluorescence and a light source in the visible region may be combined.

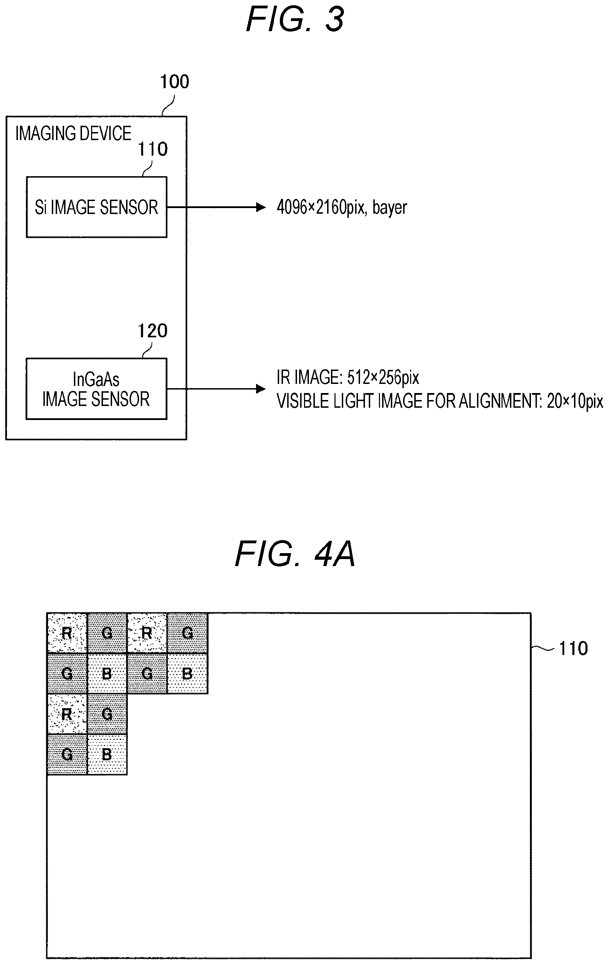

Fig. 3 is a schematic diagram illustrating an example of the number of sensor pixels of the Si image sensor 110 and the InGaAs image sensor 120. As an example, the Si image sensor 110 includes 3840 × 2160 pixels (pix), and the pixels are arrayed in a Bayer array. On the other hand, the InGaAs image sensor 120 includes 512 × 256 pixel (pix). Out of the pixels of the InGaAs image sensor 120, 20 × 10 pixels (pix) are configured as visible light pixels for alignment in order to align with visible light pixels obtained by the Si image sensor 110. The visible light pixel for alignment is to be described later. Note that, the Si image sensor 110 may be a sensor including 4096 × 2160 pixels (pix) or a high-resolution sensor including 4096 × 2160 pixels (pix) or more (for example, 7680 × 4320 pixels (pix)).

As illustrated in Fig. 1, the signal processing device 200 includes a white light image processor 202, a separating processor 204, a deformation parameter generating processor 206, an IR image processor 210, and a synthesizing processor 220. The white light image processor 202 includes a developing processor 202a and an image quality improving processor 202b. Furthermore, the IR image processor 210 includes a filling processor 212, an image quality improving processor 214, a useful information image extracting unit (image extracting unit) 216, a useful information image processor 217, an image deforming/enlarging processor (image conforming unit) 218.

### 3. Alignment and synthesis of images obtained from two image sensors

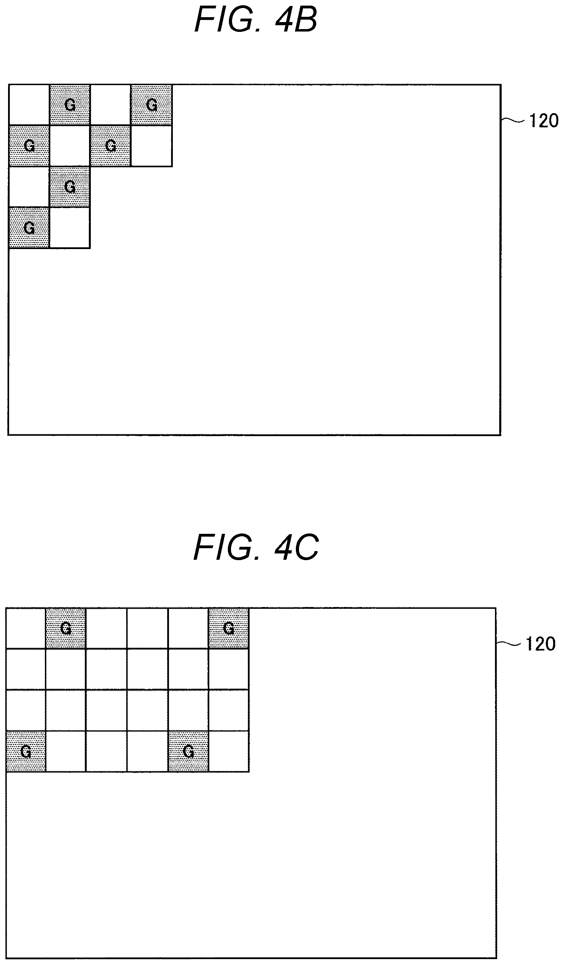

In a case of imaging a color image with one Si image sensor 110, as illustrated in Fig.4A, a Bayer method of combining each pixel with any one of color filters of three colors of red, green, and blue (RGB) is common. Figs. 4B and 4C illustrate examples in which a plurality of color filters which transmits a specific wavelength region is applied to each pixel also in the InGaAs image sensor 120, the examples in which a color filter for green used in the Si image sensor 110 is also applied to the InGaAs image sensor 120.

When the color filter for green is applied to the InGaAs image sensor 120, the color filter for green used in the Si image sensor 110 is applied to the same pixel position as that of the color filter for green of the Si image sensor 110. Therefore, the InGaAs image sensor 120 may image light transmitted through the color filter for green. Then, when an image transmitted through the color filter for green of the Si image sensor 110 and an image transmitted through the color filter for green of the InGaAs image sensor 120 are observed, the same object is observed in the same wavelength region. Therefore, correlation between the images imaged by the two image sensors of the Si image sensor 110 and the InGaAs image sensor 120 may be utilized, and the two images may be aligned on the basis of the correlation. This may be realized by the fact that the InGaAs image sensor 120 is also sensitive to the visible light wavelength region as described above. Note that, since the alignment is performed on the basis of a pixel value of the pixel in which the color filter for green is arranged, the resolution becomes higher than that in a case where color filters of other colors are used, so that the alignment may be performed with high accuracy.

Fig. 4B illustrates an example in which the color filters for green are arranged in a relatively large number of pixels in the InGaAs image sensor 120. In this case, the alignment of the Si image sensor 110 and the InGaAs image sensor 120 is focused on, and the alignment accuracy may be improved. Furthermore, Fig. 4C illustrates an example in which the number of color filters for green is decreased in the InGaAs image sensor 120 and the number of original pixels of the InGaAs image sensor 120 is increased. In this case, imaging focusing on an image quality of special light by the InGaAs image sensor 120 may be performed.

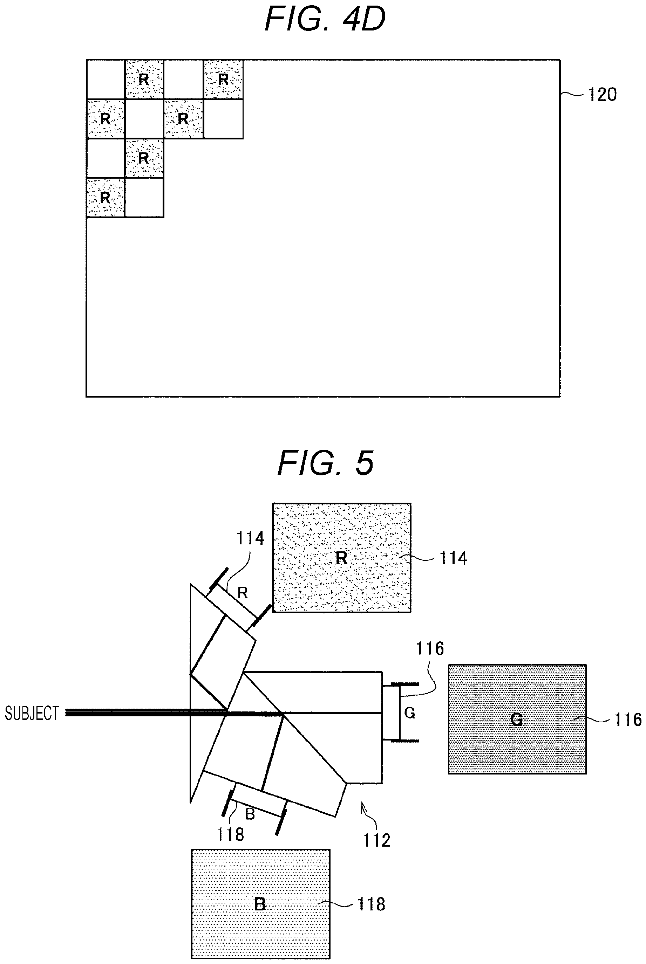

Note that the color filter applied to the InGaAs image sensor 120 may be in red or blue. In this case also, the red or blue color filter is applied to the same pixel position as that of the color filter of the same color in the Si image sensor 110. Fig. 4D illustrates an example in which the red filter is applied to the InGaAs image sensor 120.

Furthermore, since the Si image sensor 110 is also sensitive to the near-infrared region, a transmission filter for near infrared may also be applied to the same pixel position of both the Si image sensor 110 and the InGaAs image sensor 120. Therefore, it is possible to align the images of both the Si image sensor 110 and the InGaAs image sensor 120 on the basis of the pixel value obtained from the pixel transmitted through the transmission filter for near-infrared.

Note that, in this embodiment, a single-plate Bayer system which images the respective colors of RGB with a single image sensor is assumed as for the Si image sensor 110; however, the sensor is not limited to this configuration. For example, a three-plate system which uses Si image sensors 114, 116, and 118 dedicated to R, G, and B, respectively, in combination with a dichroic mirror 112 may also be employed as illustrated in Fig. 5.

### 4. Extraction of useful information image

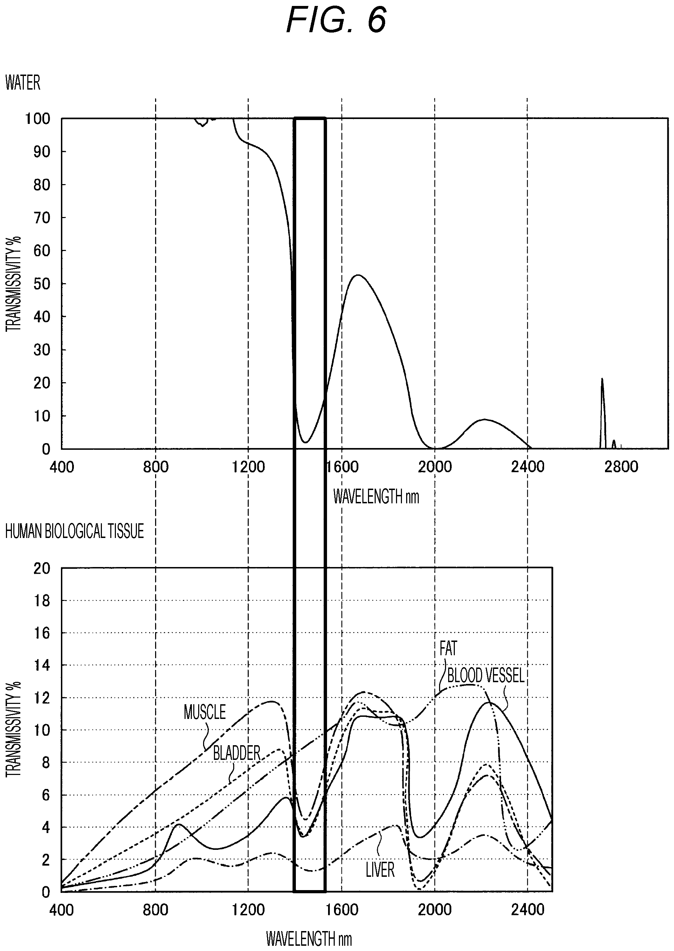

Next, a method of extracting a useful information image regarding a living body from the image imaged by the InGaAs image sensor 120 is described. Fig. 6 is a characteristic diagram illustrating transmissivity of biological tissue. Note that, the characteristic illustrated in Fig. 6 is disclosed in, for example, Japanese Patent Application Laid-Open No. 2007-75366.

In Fig. 6, a characteristic of transmissivity of water with respect to a wavelength is illustrated in an upper stage, and a characteristic of the transmissivity of the biological tissue of human with respect to the wavelength is illustrated in a lower stage. The wavelength along the abscissa corresponds between the characteristics in the upper and lower stages. As illustrated in Fig. 6, it is understood that the transmissivity of the fat is specifically higher than the transmissivity of other living tissue and water in a wavelength region from 1400 nm to 1500 nm. That is, it is possible to distinguish the fat from other tissue by combining the pixel of the InGaAs image sensor 120 with a filter which selectively transmits this wavelength region. Note that, as illustrated in Fig. 2, the Si image sensor 110 cannot image the wavelength region from 1400 to 1500 nm.

When combining the InGaAs image sensor 120 with the filter which transmits the wavelength region from 1400 nm to 1500 nm and emitting wide band light covering the near-infrared region to image, the signal value obtained through the filter is a signal of the wavelength region around 1400 nm to 1500 nm.

At that time, as illustrated in Fig. 6, the transmissivity of the tissue other than the fat is low. In other words, the tissue other than the fat has high absorbance. For this reason, the tissue other than the fat absorbs a lot of light to have a dark signal value, and the fat tissue has a bright signal value because of its low absorbance.

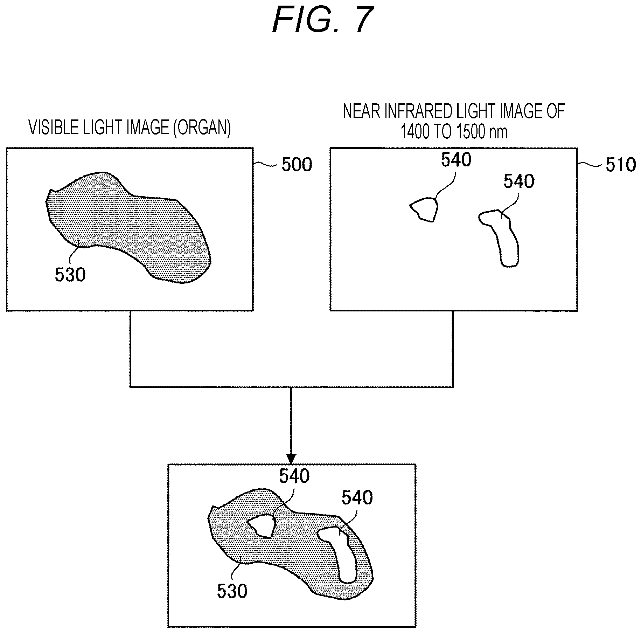

Fig. 7 is a schematic diagram illustrating an image 510 obtained by imaging while combining the InGaAs image sensor 120 with the filter which transmits the wavelength region from 1400 to 1500 nm, and a visible light image 500 obtained by imaging with the Si image sensor 110. In an example illustrated in Fig. 7, as illustrated in the visible light image 500, a state in which a specific organ 530 is imaged is illustrated. The organ 530 includes the fat tissue, but the fat tissue cannot be distinguished from the visible light image 500 obtained by imaging with the Si image sensor 110. Especially, in a case where the fat tissue is present inside the organ 530, it is difficult to distinguish or recognize the fat tissue.

On the other hand, useful information may be extracted from the image 510 of the InGaAs image sensor 120 by the method described above, and the fat tissue has the bright signal value because of its low absorbance. Therefore, a fat portion 540 in the near-infrared image obtained from the InGaAs sensor 120 is a white and bright region in the image 510 in Fig. 7. Therefore, the fat portion 540 may be extracted by extracting a bright pixel having a pixel value equal to or larger than a predetermined value from the image 510. Therefore, a living body region extracting function of extracting the region of the fat portion 540 as the useful information image may be realized. On the contrary, it is also possible to regard a region with a low pixel value, that is, a dark region in the image 510 as a region with a large moisture content.

Fig. 7 illustrates a superimposed image 520 obtained by synthesizing the visible light image 500 of the Si image sensor 110 and the useful information image extracted from the image 510 of the InGaAs image sensor 120. From the superimposed image 520, an outline and an appearance of the organ 530 may be recognized from the visible light image 500 of the Si image sensor 110, and the region and state of the fat portion 540 which cannot be distinguished from the image 500 may be distinguished from the useful information image extracted from the image 510 of the InGaAs image sensor 120. Therefore, it becomes possible to surely distinguish a range and a state of the fat portion 540 generated in the organ 530. Therefore, in a case of performing surgical operation on the organ 530, the surgical operation may be performed in consideration of the position and state of the fat portion 540.

Furthermore, in a case where the filter which transmits the wavelength region from 1400 nm to 1500 nm is used in the InGaAs image sensor 120, the transmissivity of the fat is high in this wavelength region and the light of the fat portion 540 is transmitted, but the light of other tissue is not transmitted. For this reason, in a case where the fat portion 540 overlaps with another tissue, it is possible to observe a state in which the fat portion 540 is made transparent.

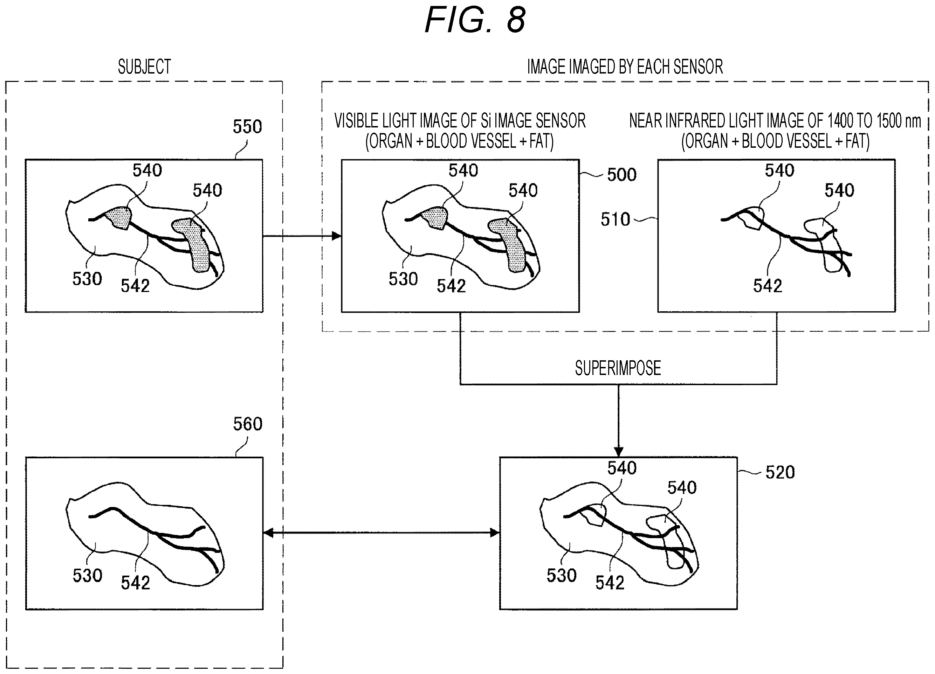

Fig. 8 is a schematic diagram illustrating an example in which a blood vessel 542 may be recognized through the fat portion 540 in a case where a subject 550 in which the fat portion 540 covers the organ 530 including the blood vessel 542 is imaged. In the image 500 obtained by imaging the subject 550 by the Si image sensor 110, the organ 530, the fat portion 540, and the blood vessel 542 are imaged; however, since the fat portion 540 is formed on the blood vessel 542, a state of the blood vessel 542 under the fat portion 540 cannot be distinguished.

On the other hand, in the image 510 obtained by imaging while combining the InGaAs image sensor 120 with the filter which transmits the wavelength region from 1400 to 1500 nm, the transmissivity of light in the fat portion 540 is high and the transmissivity of light in the blood vessel 542 is low, so that the light penetrates the fat portion 540 and the blood vessel 542 is seen through.

Therefore, in the superimposed image 520 obtained by synthesizing the visible light image 500 of the Si image sensor 110 and the image 510 of the InGaAs image sensor 120, the state of the blood vessel 542 through the fat portion 540 may be observed in detail. Furthermore, since the superimposed image 520 includes the visible light image 500, color reproduction is natural, and visibility and recognizability may be improved. Note that, in the superimposed image 520 in Fig. 8 also, it is desirable to superimpose the useful information image obtained by extracting the fat portion 540 and the blood vessel 542 from the image 510 of the InGaAs image sensor 120 on the visible light image 500.

In Fig. 8, a subject 560 without the fat portion 540 is illustrated for comparison. The subject 560 is the same as the subject 550 except that there is no fat portion 540. In the superimposed image 520, since the blood vessel 542 may be visually recognized through the fat portion 540, it is possible to obtain an image which maintains normal color reproduction while making the fat portion 540 transparent. Therefore, as is apparent from comparison between the subject 550 and the superimposed image 520, it is possible to obtain the superimposed image 520 similar to that in a case where the subject 560 without the fat portion 540 is imaged as the visible light image.

For example, if the blood vessel 542 which cannot be visually recognized due to the fat portion 540 is present in a surgical scene, it is assumed that the blood vessel 542 is erroneously excised. In such a case, by using the superimposed image 520 according to this embodiment, the blood vessel 542 may be observed through the fat portion 540, so that a situation in which the blood vessel 542 is erroneously excised during the surgical operation may be certainly suppressed.

When generating the superimposed image 520, it is also possible to generate the superimposed image 520 by making the IR image a monochrome image, making the color thereof an arbitrary single color, and alpha blending the same with the visible light image. In monochromatization, green or blue which hardly exists in the human body is preferably selected.

Furthermore, in the above-described example, an example of synthesizing the visible light image 500 of the Si image sensor 110 and the image 510 of the InGaAs image sensor 120 is described; however, the two images may be simultaneously displayed in one display by a side-by-side (SideBySide) or picture-in-picture (PictureInPicture) method. Furthermore, the images may be displayed on two displays. Furthermore, not only 2D display but also stereo 3D display may be performed. Moreover, a human wearable display device such as a head-mounted display may be displayed as the display device 400.

### 5. Process performed in surgical imaging system according to this embodiment

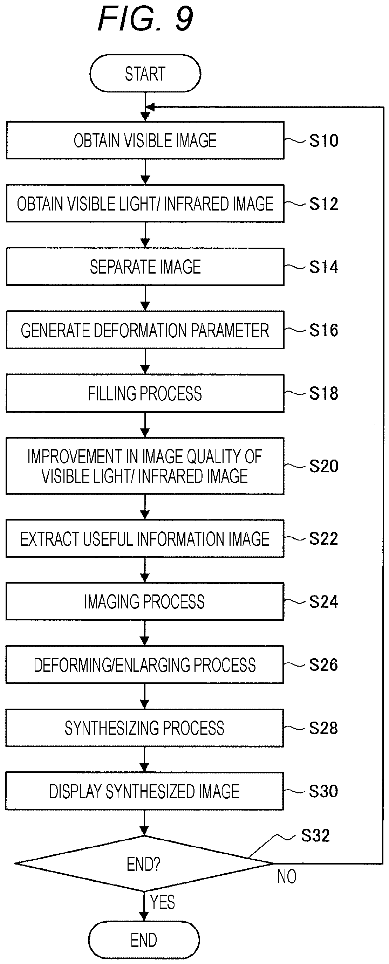

Next, a process performed by the surgical imaging system 1000 according to this embodiment is described with reference to the block diagram in Fig. 1 on the basis of a flowchart in Fig. 9. The process in Fig. 9 is mainly performed by the signal processing device 200. First, at step S10, the visible light image imaged by the Si image sensor 110 is obtained. The visible light image is subjected to a developing process by the developing processor 202a in the white light image processor 202, and subjected to an image quality improving process by the image quality improving processor 202b.

At next step S12, the visible light/infrared light image imaged by the InGaAs image sensor 120 is obtained. At next step S14, the separating processor 204 separates the visible light/infrared light image into the IR image and the visible light image for alignment. Here, the IR image is an image including the pixel other than the pixel in which the color filter for green is arranged illustrated in Fig. 4B. Furthermore, the visible light image for alignment is an image including the pixel in which the color filter for green is arranged illustrated in Fig. 4B. Note that, the IR image has the resolution lower than that of the visible light image imaged by the Si image sensor 110, and the visible light image for alignment has the resolution lower than that of the IR image.

At next step S16, the deformation parameter generating processor 206 compares the visible light image imaged by the Si image sensor 120 with the visible light image for alignment separated by the separating processor 204. Then, the deformation parameter generating processor 206 generates a deformation parameter for deforming or enlarging the visible light/infrared light image obtained by the InGaAs image sensor 120 in accordance with the visible light image imaged by the Si image sensor 120.

Since the Si image sensor 110 and the InGaAs image sensor 120 are assumed to be different in resolution and angle of view depending on lens characteristics thereof, an image size is appropriately changed as the signal processing before superimposed display of the visible light image and the visible light/infrared image is performed. For example, in a case where the Si image sensor 110 has 4K resolution (3840 × 1080) and the InGaAs image sensor 120 has HD resolution (1920 × 1080) lower than that, the resolution of the visible light/infrared image imaged by the InGaAs image sensor 120 is converted to the resolution corresponding to 4K resolution (up conversion) without changing an aspect ratio thereof. The deformation parameter generating processor 206 generates the deformation parameter for changing the image size in such a manner.

Furthermore, the alignment and distortion correction of the images may be performed as the signal processing before the superimposed display of the visible light image and the visible light/infrared light image is performed. For example, in a case of performing the frame sequential imaging by time division, if the subject or the camera moves, positional displacement might occur between the two images. Furthermore, in a case of simultaneously imaging by the Si image sensor 110 and the InGaAs image sensor 120, the positional displacement according to positions of both the sensors and an optical system occurs. Alternatively, a difference in image size or distortion between the Si image sensor 110 and the InGaAs image sensor 120 might occur due to differences in axial chromatic aberration for each wavelength and in lens characteristic. The deformation parameter generating processor 206 generates the deformation parameter in order to perform the alignment and distortion correction of such images. In a case where the subject or camera moves in the frame sequential imaging by time division, it is possible to compare the visible light image of the Si image sensor 110 with the visible light image for alignment of the InGaAs image sensor 120 and perform block matching, thereby performing the alignment. Furthermore, the positional displacement according to the positions of both the sensors and the optical system, and the difference in axial chromatic aberration for each wavelength and in lens characteristic may be obtained in advance from specifications of the imaging device 100 and both the sensors.

Note that it is also possible to create a depth map by parallax estimation using image data in the same position of the visible light image and the visible light/infrared light image after the alignment is performed.

At next step S18, the filling processor 212 performs a process of filling the pixel value of the visible light image for alignment on the IR image separated by the separating processor 204. Specifically, a process of interpolating the pixel value of the pixel in which the color filter for green is arranged illustrated in Fig. 4B with the pixel values of surrounding pixels is performed.

At next step S20, the image quality improving processor 214 performs a process of improving the image quality of the IR image subjected to the filling process by the filling processor 212. The image quality improving processor 214 improves the image quality of the IR image imaged by the InGaAs image sensor 120 by the signal processing on the basis of the image information of the visible light image imaged by the Si image sensor 110. For example, the image quality improving processor 214 estimates a PSF blur amount (PSF) between the visible light image and the IR image using the visible image imaged by the Si image sensor 110 as a guide. Then, by removing the blur of the IR image so as to conform the blur amount of the visible light image, a contrast of the IR image is improved and the image quality is improved.

At next step S22, the useful information image extracting unit 216 extracts the useful information image regarding the living body from the IR image subjected to the image quality improving process. The useful information image is, for example, image information indicating a region of the fat portion 540 in the IR image as illustrated in Figs. 7 and 8. In a case where the visible light image and the IR image are simply synthesized, there is a case in which the fat portion 540 is not displayed with emphasis, so that a process of extracting the region of the fat portion 540 as the useful information image and removing other regions is performed. Therefore, the region of the fat portion 540 may be displayed with emphasis after the synthesis with the visible light image.

At next step S24, the useful information image processor 217 performs an imaging process on the useful information image. Here, for example, the region of the fat portion 540 corresponding to the useful information image is colored in a color (green, blue and the like) which does not exist in the human body. Therefore, the region of the fat portion 540 may be displayed with emphasis after the synthesis with the visible light image.

At next step S26, the image deforming/enlarging processor 218 applies the deformation parameter to the useful information image to perform a deforming/enlarging process of the useful information image. Therefore, the position and size of the visible light image imaged by the Si image sensor 110 conform to those of the useful information image. Furthermore, by applying the deformation parameter, the axial chromatic aberration for each wavelength and the distortion of the lens characteristic are corrected to the same level in the visible light image imaged by the Si image sensor 110 and the useful information image. At next step S28, the synthesizing processor 220 synthesizes the visible light image processed by the white light image processor 202 and the useful information image processed by the IR image processor 210. Information of the synthesized image (superimposed image) generated by the synthesis is transmitted from the signal processing device 200 to the transmitting device 300 and further transmitted to the display device 400.

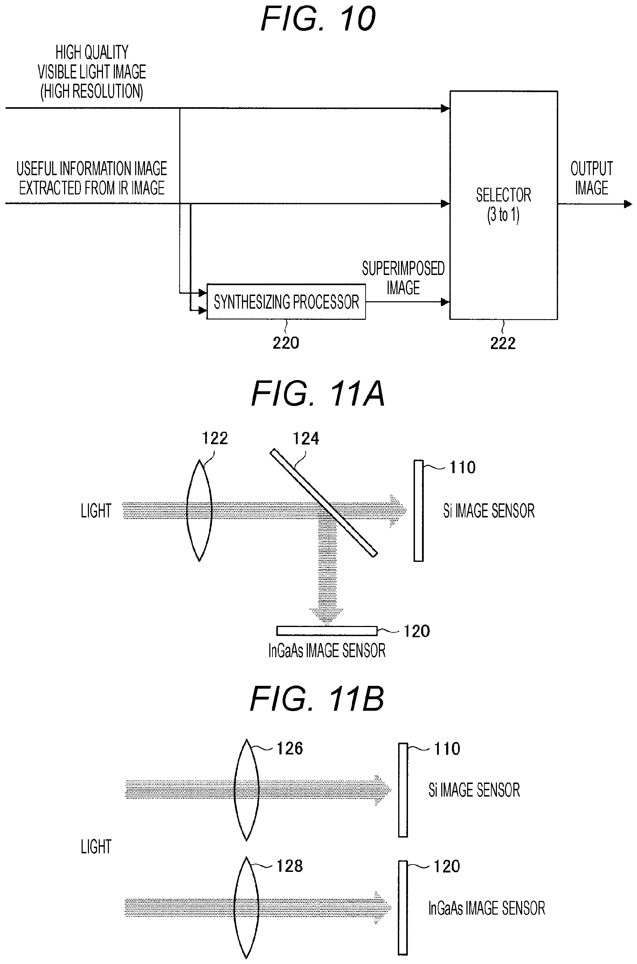

Fig. 10 is a schematic diagram illustrating the synthesizing processor 220 and a peripheral configuration thereof. As illustrated in Fig. 10, a selector 222 may be provided on a subsequent stage of the synthesizing processor 220. To the selector 222, in addition to the synthesized image synthesized by the synthesizing processor 220, an image before the synthesis, that is, the visible light image output from the white light image processor 202 and the useful information image output from the IR image processor 210 are input.

From the selector 222, any one of the synthesized image synthesized by the synthesizing processor 220, the visible light image processed by the white light image processor 202, or the useful information image processed by the IR image processor 210 is selected to be output to the transmitting device 300. Therefore, any one of the synthesized image, the visible light image, or the useful information image is transmitted from the transmitting device 300 to the display device 400, so that these images may be displayed on the display device 400. Note that switching of the images by the selector 222 is performed when operation information by a user is input to the selector. In a case where the synthesized image is displayed, the information obtained from the Si image sensor 110 and the information obtained from the InGaAs image sensor 120 may be visually recognized at once, so that the information may be obtained most efficiently.

At next step S30, the display device 400 displays the image information transmitted from the transmitting device 300. At next step S32, it is determined whether or not to finish the process. In a case where the process is not finished, the procedure returns to step S10 to perform the subsequent process.

### 6. Configuration example of optical system

Figs. 11A to 11C are schematic diagrams illustrating an optical system of the imaging device 100. As the optical system, as illustrated in Fig. 11A, a "single-eye two-plate system" in which light is introduced from one opening through a lens 122 to be guided to the Si image sensor 110 and the InGaAs image sensor 120 with a splitter 124 arranged inside the imaging device 100 may be employed. In this case, since chromatic aberration on an optical axis varies depending on the wavelength, it is desirable to appropriately design the positions of the lens 122, the Si image sensor 110, and the InGaAs image sensor 120 in order to reduce an influence.

Furthermore, as illustrated in Fig. 11B, a "two-lens two-plate system" in which light is introduced from two openings through lenses 126 and 128 to be guided to the Si image sensor 110 and the InGaAs image sensor 120, respectively, may be employed. In this case, parallax due to the difference in position between the two openings is appropriately corrected when the superimposed image is generated.

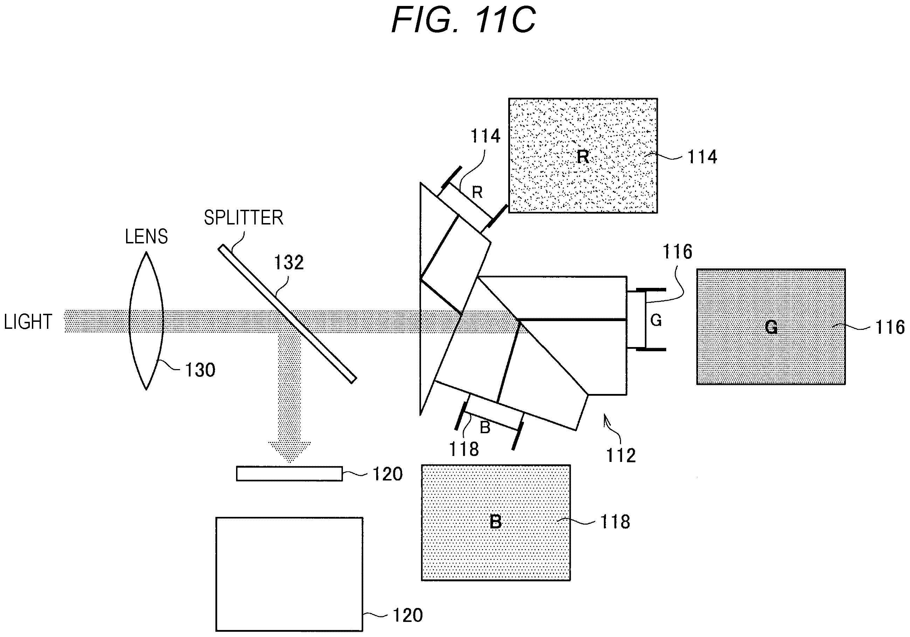

Furthermore, Fig. 11C illustrates an example in which a three-plate system including the dichroic mirror 112 and using the dedicated Si image sensors 114, 116, and 118 for R, G, and B, respectively as in Fig. 5 is employed. In this case, light is introduced from one opening through a lens 130, the light transmitted through the lens 130 enters a splitter 132, and the light dispersed by the splitter 132 is emitted to each of the dichroic mirror 112 and the InGaAs image sensor 120.

As described above, according to this embodiment, it is possible to improve the visibility of the blood vessels and fat regions difficult to determine only with the normal visible light image. Furthermore, it becomes possible to improve a sense of resolution of the image imaged by the InGaAs image sensor 120. Moreover, simultaneous observation becomes possible by superimposed display of the images imaged by the InGaAs image sensor 120 and the Si image sensor 110.

Although the preferred embodiment of the present disclosure is described above in detail with reference to the attached drawings, the technical scope of the present disclosure is not limited to such examples. It is clear that one of ordinary skill in the technical field of the present disclosure may conceive of various modifications and corrections within the scope of the technical idea recited in claims and it is understood that they also naturally belong to the technical scope of the present disclosure.

Furthermore, the effects described in this specification are merely illustrative or exemplary, and are not limiting. That is, the technology according to the present disclosure may exhibit other effects obvious to those skilled in the art from the description of this specification together with or in place of the effects described above.

Note that, the following configuration also belongs to the technical scope of the present disclosure.
(1) A surgical imaging system including:
   a first image sensor that has light receiving sensitivity in a wavelength region of visible light and images a surgical site;
   a second image sensor that has light receiving sensitivity in a wavelength region of visible light and near-infrared light and images the surgical site; and
   a signal processing device that performs a process for displaying a first image imaged by the first image sensor and a second image imaged by the second image sensor.
(2) The surgical imaging system according to (1) described above, in which resolution of the first image sensor is higher than resolution of the second image sensor.
(3) The surgical imaging system according to (1) or (2) described above,
   in which the first image sensor includes a color filter in a predetermined color arranged for each pixel, and
   the second image sensor includes a color filter in the same color as the color of the color filter in a pixel position corresponding to a pixel position of the color filter of the first image sensor.
(4) The surgical imaging system according to (3) described above, in which the predetermined color is green.
(5) The surgical imaging system according to any one of (1) to (4) described above, in which the first image sensor is an image sensor including Si and has resolution of 3840 × 2160 pixels or more.
(6) The surgical imaging system according to any one of (1) to (5) described above, in which the second image sensor is an image sensor including InGaAs.
(7) The surgical imaging system according to (3) described above, in which the signal processing device includes an image conforming unit that conforms the first image to the second image on the basis of a pixel value obtained through the color filter of the first image sensor and a pixel value obtained through the color filter of the second image sensor.
(8) The surgical imaging system according to (3) described above, in which the signal processing device includes a filling processor that calculates a pixel value in a state in which the color filter is not arranged in the pixel position in which the color filter is provided of the second image sensor.
(9) The surgical imaging system according to any one of (1) to (8) described above, in which the signal processing device includes a synthesizing processor that synthesizes the first image and the second image.
(10) The surgical imaging system according to any one of (1) to (9) described above, in which the signal processing device includes an image quality improving processor that improves an image quality of the second image on the basis of the first image.
(11) The surgical imaging system according to any one of (1) to (10) described above, in which the signal processing device includes an image extracting unit that extracts a specific region from the second image.
(12) The surgical imaging system according to (11) described above,
   in which the second image sensor includes a filter that transmits light in a predetermined wavelength region, and
   the image extracting unit extracts the specific region on the basis of a pixel value obtained through the filter.
(13) The surgical imaging system according to (12) described above, in which the predetermined wavelength region is a wavelength region not shorter than 1300 nm and not longer than 1400 nm.
(14) The surgical imaging system according to (11) described above, in which the signal processing device includes an image processor that assigns a predetermined color to the specific region.
(15) The surgical imaging system according to (14) described above, in which the predetermined color is green or blue.
(16) The surgical imaging system according to any one of (1) to (15) described above, in which the first image sensor and the second image sensor image fat or a blood vessel in a human body.
(17) A signal processing device of a surgical image, performing a process for synthesizing to display a first image imaged by a first image sensor that has light receiving sensitivity in a wavelength region of visible light and images a surgical site and a second image imaged by a second image sensor that has light receiving sensitivity in a wavelength region of visible light and near-infrared light and images the surgical site.

### REFERENCE SIGNS LIST

- 100: Imaging device
- 110: Si image sensor
- 120: InGaAs image sensor
- 200: Signal processing device
- 212: Filling processor
- 214: Image quality improving processor
- 216: Useful information image extracting unit
- 217: Useful information image processor
- 218: Image deforming/enlarging processor
- 220: Synthesizing processor
- 1000: Surgical imaging system

## Claims

1. A surgical imaging system comprising:
a first image sensor that has light receiving sensitivity in a wavelength region of visible light and images a surgical site;
a second image sensor that has light receiving sensitivity in a wavelength region of visible light and near-infrared light and images the surgical site; and
a signal processing device that performs a process for displaying a first image imaged by the first image sensor and a second image imaged by the second image sensor.

2. The surgical imaging system according to claim 1, wherein resolution of the first image sensor is higher than resolution of the second image sensor.

3. The surgical imaging system according to claim 1,
wherein the first image sensor includes a color filter in a predetermined color arranged for each pixel, and
the second image sensor includes a color filter in a same color as the color of the color filter in a pixel position corresponding to a pixel position of the color filter of the first image sensor.

4. The surgical imaging system according to claim 3, wherein the predetermined color is green.

5. The surgical imaging system according to claim 1, wherein the first image sensor is an image sensor including Si and has resolution of 3840 × 2160 pixels or more.

6. The surgical imaging system according to claim 1, wherein the second image sensor is an image sensor including InGaAs.

7. The surgical imaging system according to claim 3, wherein the signal processing device includes an image conforming unit that conforms the first image to the second image on a basis of a pixel value obtained through the color filter of the first image sensor and a pixel value obtained through the color filter of the second image sensor.

8. The surgical imaging system according to claim 3, wherein the signal processing device includes a filling processor that calculates a pixel value in a state in which the color filter is not arranged in the pixel position in which the color filter is provided of the second image sensor.

9. The surgical imaging system according to claim 1, wherein the signal processing device includes a synthesizing processor that synthesizes the first image and the second image.

10. The surgical imaging system according to claim 1, wherein the signal processing device includes an image quality improving processor that improves an image quality of the second image on a basis of the first image.

11. The surgical imaging system according to claim 1, wherein the signal processing device includes an image extracting unit that extracts a specific region from the second image.

12. The surgical imaging system according to claim 11,
wherein the second image sensor includes a filter that transmits light in a predetermined wavelength region, and
the image extracting unit extracts the specific region on a basis of a pixel value obtained through the filter.

13. The surgical imaging system according to claim 12, wherein the predetermined wavelength region is a wavelength region not shorter than 1300 nm and not longer than 1400 nm.

14. The surgical imaging system according to claim 11, wherein the signal processing device includes an image processor that assigns a predetermined color to the specific region.

15. The surgical imaging system according to claim 14, wherein the predetermined color is green or blue.

16. The surgical imaging system according to claim 1, wherein the first image sensor and the second image sensor image fat or a blood vessel in a human body.

17. A signal processing device of a surgical image, performing a process for synthesizing to display a first image imaged by a first image sensor that has light receiving sensitivity in a wavelength region of visible light and images a surgical site and a second image imaged by a second image sensor that has light receiving sensitivity in a wavelength region of visible light and near-infrared light and images the surgical site.
